# EUROPEAN PATENT SPECIFICATION

(11) **EP 1 935 332 B1**
(45) Date of publication and mention of the grant of the patent: **01.10.2014**
(21) Application number: 07255010.6
(22) Date of filing: 21.12.2007
(51) Int. Cl.: A61B 5/00, A61B 8/12, A61B 18/14

(54) **Real-Time optoacoustic monitoring with electrophysiologic catheters**
Optoakustische Echtzeitüberwachung mit elektrophysiologischen Kathetern
Surveillance opto-acoustique en temps réel avec cathéters électrophysiologiques

(30) Priority: 22.12.2006 US 644312
(43) Date of publication of application: 25.06.2008
(73) Proprietor: Biosense Webster, Inc., Diamond Bar, CA 91765 (US)
(72) Inventor: Sharareh, Shiva, Laguna Niguel CA 92677 (US); Lieber, Chad A., Chino Hills CA 91709 (US)
(74) Representative: Small, Gary James

(56) References cited:
- WO-A-2004/000112
- WO-A-2004/000148
- US-A1- 2002 002 372
- US-B1- 6 200 310
- ROOME K A ET AL: "Towards a sideways looking intravascular laser-ultrasound probe" SENSORS AND ACTUATORS A, ELSEVIER SEQUOIA S.A., LAUSANNE, CH, vol. 76, no. 1-3, 30 August 1999 (1999-08-30), pages 197-202, XP004184437 ISSN: 0924-4247
- HENRICHS P M ET AL: "Atherosclerotic plaque characterization with optoacoustic imaging" PROCEEDINGS OF THE SPIE - THE INTERNATIONAL SOCIETY FOR OPTICAL ENGINEERING SPIE-INT. SOC. OPT. ENG USA, vol. 5697, no. 1, 2005, pages 217-223, XP002482197 ISSN: 0277-786X
- KROTOV EUGENE V ET AL: "Detection of thermal acoustic radiation from laser-heated deep tissue" APPLIED PHYSICS LETTERS, AIP, AMERICAN INSTITUTE OF PHYSICS, MELVILLE, NY, vol. 81, no. 21, 18 November 2002 (2002-11-18), pages 3918-3920, XP012032553 ISSN: 0003-6951

## Description

### FIELD OF INVENTION

The present invention relates to electrophysiologic catheters, and in particular to laser-optoacoustic electrophysiologic catheters for monitoring tissue and lesion assessment.

### BACKGROUND

For certain types of minimally invasive medical procedures, real time information regarding the condition of the treatment site within the body is unavailable. This lack of information inhibits the clinician when employing a catheter to perform a procedure. An example of such procedures is tumor and disease treatment in the liver and prostate. Yet another example of such a procedure is cardiac ablation used to treat atrial fibrillation. This condition in the heart causes abnormal electrical signals to be generated in the endocardial tissue resulting in irregular beating of the heart.

The most frequent cause of cardiac arrhythmias is an abnormal routing of electricity through the cardiac tissue. In general, most arrhythmias are treated by ablating suspected centers of this electrical misfiring, thereby causing these centers to become inactive. Successful treatment, then, depends on the location of the ablation within the heart as well as the lesion itself. For example, when treating atrial fibrillation, an ablation catheter is maneuvered into the right or left atrium where it is used to create ablation lesions in the heart. These lesions are intended to stop the irregular beating of the heart by creating non-conductive barriers between regions of the atria that halt passage through the heart of the abnormal electrical activity.

The lesion should be created such that electrical conductivity is halted in the localized region (transmurality), but care should be taken to prevent ablating adjacent tissues. Furthermore, the ablation process can also cause undesirable charring of the tissue and localized coagulation, and can evaporate water in the blood and tissue leading to steam pops.

Currently, lesions are evaluated following the ablation procedure, by positioning a mapping catheter in the heart where it is used to measure the electrical activity within the atria. This permits the physician to evaluate the newly formed lesions and determine whether they will function to halt conductivity. It if is determined that the lesions were not adequately formed, then additional lesions can be created to further form a line of block against passage of abnormal currents. Clearly, post ablation evaluation is undesirable since correction requires additional medical procedures. Thus, it would be more desirable to evaluate the lesion as it is being formed in the tissue.

A known method for evaluating lesions as they are formed is to measure electrical impedance. Biochemical differences between ablated and normal tissue can result in changes in electrical impedance between the tissue types. Although impedance is routinely monitored during electrophysiologic therapy, it is not directly related to lesion formation. Measuring impedance merely provides data as to the location of the tissue lesion but does not give qualitative data to evaluate the effectiveness of the lesion.

Another approach is to measure the electrical conductance between two points of tissue. This process, known as lesion pacing, can also determine the effectiveness of lesion therapy. This technique, however, measures the success or lack thereof from each lesion, and yields no real-time information about the lesion formation.

In a broader sense, ultrasonic imaging is also known for detecting abnormalities in soft tissue organs with acoustic boundaries. But tissues can be acoustically homogenous and therefore undetectable by ultrasound imaging. Similar limitations are posed by optical imaging based on time-resolved or phase resolved detection of diffusely reflected light pulses or photon density waves.

Laser optoacoustic technology can offer advantages over the aforementioned technologies. Improvement in sensitivity, spatial resolution and interpretation of images is possible with suitable manipulation of (1) short-pulse laser irradiation to generate transient stress waves under conditions of temporal stress confinement, where such irradiations provide large amplitude of generated stress with profiles resembling that of light distribution in tissues to yield sharp images with accurate localization; (2) time-resolved detection of stress profile for obtaining diagnostic information from the temporal profile of generated stress wave; and (3) use of wide-band piezoelectric detectors to correctly reproduce stress profiles to obtain spatial resolution of tomography. However, application of this technology in vivo, and particularly in vivo endocardial and epicardial applications, has been limited due to various factors, including space constraints and integration of the equipment to provide irradiation and detection of optoacoustic data.

WO2004/000148A2 discloses an apparatus for forming a hole in a region of heart muscle wall and measuring the depth of the holes so formed. WO2004/000112A2 discloses a tissue viability monitor for determining the viability of biological tissue. Roome, K.A. et al., "Towards a sideways looking intravascular laser-ultrasound probe" discloses a device for laser ultra-sound imaging of tissue.
There is a need for an integrated electrophysiologic catheter capable of monitoring tissue and performing lesion assessment, especially for endocardial and epicardial tissue, in real-time using optoacoustic technology for improved sensitivity and spatial resolution.

### SUMMARY OF THE INVENTION

The present invention recognizes that light delivered in sufficiently short pulse widths is selectively absorbed by tissue elements and surrounding medium (blood), and is converted to heat. This heat produces an acoustic wave which can be detected by an acoustic sensor. A delay in receive time of the acoustic wave is proportional to the distance of elements generating the acoustic wave from the light delivery optics, and can be used to determine tissue thickness. To that end, optoacoustic imaging employs non-resonant acoustic frequencies which result from optical absorption properties of materials within the field of view of the light delivery optics. As such, the signal output has greater sensitivity to materials with different optical absorption properties, such as those between tissue and blood or air. It is therefore possible to obtain high resolution imaging of biological tissue through blood, with an operable range up to several centimeters (as determined by wavelength, optical absorption, and acoustic sensor size). Such imaging can be particularly advantageous during and concurrently with ablation for visualization of lesion formation.

The present invention is directed to a system for opto-acoustic tissue and lesion assessment in real time on lesion width and, potentially, one or more of the following tissue characteristics: tissue thickness, lesion progression, steam pop, and char formation. The system includes an RF ablation element, laser delivery means, and an acoustic sensor. These elements work by irradiating tissue undergoing ablation treatment to create acoustic waves that have a temporal profile which can be recorded and analyzed by acoustic sampling hardware for reconstructing a cross-sectional aspect of the irradiated tissue. In accordance with the present invention, the RF ablation element , laser delivery means and acoustic sensor are configured to interact with a tissue surface from a common orientation; that is, these components are each generally facing the tissue surface such that the direction of irradiation and the direction of acoustic detection are generally opposite to each other, where the stress waves induced by the laser-induced heating of the tissue below the surface are reflected back to the tissue surface.

In a more detailed example, the system includes a catheter having an integrated distal tip section that is configured for irradiation and acoustic detection, an electronic scope and a processor. Advantageously, tissue that is heated by the irradiation from the catheter tip section produces an acoustic wave that is detected by a acoustic detector which generates a signal representative of a tissue characteristic which is received by an electronic scope to record a temporal profile of the acoustic wave. The processor uses the temporal profile to reconstruct a cross-sectional aspect of the tissue.

The present invention is directed to a catheter for opto-acoustic tissue assessment in real time. In one embodiment, the catheter has a catheter body and a distal tip section that is configured for irradiation and acoustic detection, wherein a tissue is heated by the irradiation to produce an acoustic wave that is detected by an acoustic detector mounted on the tip section and the acoustic detector generates a signal representative of a tissue characteristic. In a more detailed embodiment, the catheter is configured for use with cardiac tissue and the tip section is configured for RF ablation. Moreover, the irradiation emitted by the catheter may be a laser pulse, and the tissue of interest is a lesion resulting from RF ablation.

The present invention is designed to use optoacoustic technology in conjunction with RF ablation. To that end, the light used to heat the tissue is generally not affected by the portion of the electromagnetic radiation used for ablation. The spectral window for use in the present invention is about 400nm to 2000nm, preferably 700nm and 1100nm, determined by the absorption band(s) of the contrast species of interest.

### BRIEF DESCRIPTION OF THE DRAWINGS

These and other features and advantages of the present invention will be better understood by reference to the following detailed description when considered in conjunction with the accompanying drawings wherein:

FIG. 1 illustrates an embodiment of an opto-acoustic ablation system in accordance with the present invention.

FIG. 1A illustrates another embodiment of an opto-acoustic ablation system in accordance with the present invention.

FIG. 1B is a detailed illustration of the tip section of a catheter for use in an opto-acoustic ablation system in accordance with the present invention depicting application of such tip section to tissue.

FIG. 2A is a side cross-sectional view of an embodiment of a catheter according to the present invention, including the junction between a catheter body and an intermediate section, taken along a first diameter.

FIG. 2B is a side cross-sectional view of an embodiment of a catheter according to the invention, including the junction between a catheter body and an intermediate section, taken along a second diameter generally perpendicular to the first diameter of FIG. 2A.

FIG. 3A is a side cross-sectional view of an embodiment of a catheter according to the invention, including a junction between a plastic housing and a tip electrode, taken along a first diameter.

FIG. 3B is a side cross-sectional view of an embodiment of a catheter according to the invention, including a junction between a plastic housing and a tip electrode, taken near a second diameter generally perpendicular to the first diameter of FIG. 3A;

FIG. 3C is a longitudinal cross-sectional view of an embodiment of the intermediate section of FIGS. 2A and 2B.

FIG. 3D is a side cross-sectional view an embodiment of a catheter according to the invention, including a junction between a plastic housing and a tip electrode, taken along line 3D--3D in FIG. 4.

FIG. 4 is a longitudinal cross-sectional view of an embodiment of the tip electrode of FIGS. 3A and 3B.

FIG. 5 is a distal end view of an embodiment of a tip electrode.

FIG. 5A is a distal end view of another embodiment of a tip electrode.

FIG. 6A is a side cross-sectional view of an embodiment of an irrigated catheter according to the present invention, including the junction between a catheter body and an intermediate section, taken along a first diameter.

FIG. 6B is a side cross-sectional view of an embodiment of an irrigated catheter according to the invention, including the junction between a catheter body and an intermediate section, taken along a second diameter generally perpendicular to the first diameter of FIG. 6A.

FIG. 7 is a side cross-sectional view of an embodiment of a catheter according to the invention, including a junction between a plastic housing and an intermediate section.

FIG. 8 is a longitudinal cross-sectional view of an embodiment of the intermediate section of FIGS. 6A and 6B.

FIG. 9 is a longitudinal cross-sectional view of an embodiment of the tip electrode of FIGS. 6A and 6B.

### DETAILED DESCRIPTION OF THE INVENTION

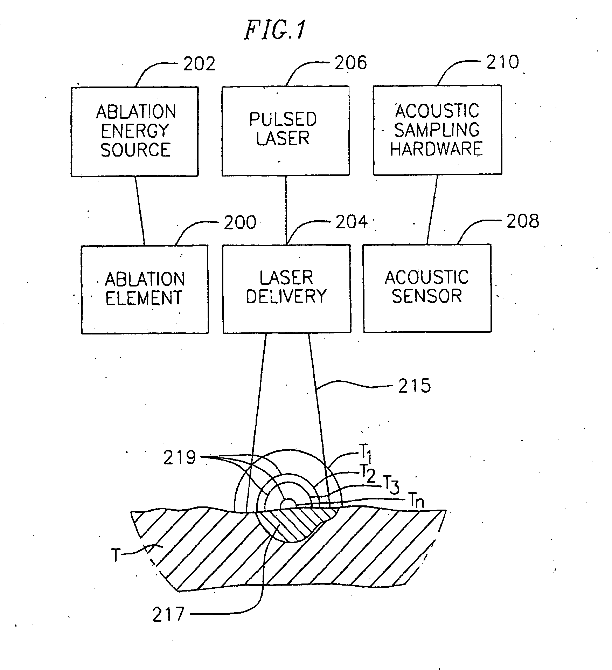

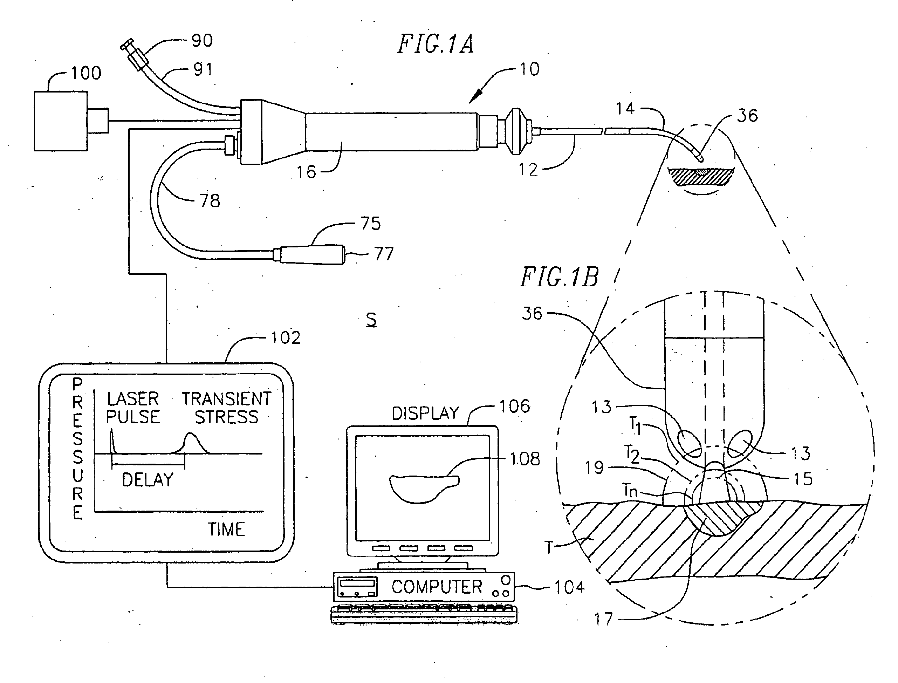

FIG. 1 illustrates an embodiment of a system S for laser optoacoustic monitoring to provide real time assessment of lesion formation, tissue status, and tissue morphology. Tissue T is subjected to RF ablation by an ablation element 200 that is energized by an ablation energy source 202 to form lesion 217. A laser delivery means 204 irradiates the lesion 217 and surrounding tissue within its field of view 215 to stimulate pressure waves 219 (with different delay times T1, T2... Tn) which are detected by acoustic transducers 208 for imaging the lesion against the surrounding tissue. The laser delivery means can include a fiber optic cable housed in a catheter that is equipped solely or primarily for irradiation, or an integrated catheter as described further below. As understood by one of ordinary skill in the art, the imaging provided by the present invention is based on contrast provided by differential absorption. To that end, a pulsed laser light source 206 drives the laser delivery means 204 to slightly but quickly heat the tissue and the lesion within an irradiation field of view of the laser delivery demeans. This heating causes microscopic expansion in the lesion and surrounding tissue, which have different optical absorptions, to generate the pressure waves 219 with discernible stress profiles that propagate outwardly. An acoustic sensor 208 detects the emitted pressure waves, including the time delays T1-Tn, and converts the stress profile into electrical signals that are received by acoustic sampling hardware 210 for reconstructing a cross-sectional representation of the lesion. And, where the delay in the receive time of the acoustic waves is proportional to the distance of the sources generating the acoustic waves from the laser delivery means 204, the detected signals can be used to determine tissue thickness, lesion progression, lesion width, and other assessment features in real time. Moreover, by employing non-resonant acoustic frequencies which are the result of optical absorption properties of the various materials within the irradiation field of view of the laser delivery means, the resulting signal tends to have a much higher sensitivity to materials with different optical absorption properties, such as those between tissue in various states of ablation, tissue and blood.

In a more detailed embodiment of FIG. 1A, a catheter-based system S for real-time laser optoacoustic monitoring is illustrated. Endo- or epi-cardial tissue T is subjected to RF ablation by a catheter 10 having a tip section 36 adapted for RF ablation in creating a lesion 17. To that end, the catheter tip section 36 has an integrated structure (see FIG. 1B) from which radiation 15 is emitted to heat the lesion 17 and surrounding tissue and stimulate pressure waves 19 (with different delay times T1, T2...Tn) which are detected by acoustic transducers 13 for imaging the lesion against the surrounding tissue. A light source 100 provides pulsed irradiation that is delivered to the catheter tip 36 to slightly but quickly heat the tissue and the lesion within an irradiation field of view of the tip section 36. Similarly, this heating causes microscopic expansion in the lesion and surrounding tissue, which have different optical absorptions, to generate pressure waves with discernible stress profiles that propagate outwardly. The transducers 13, which may include piezoelectric transducers, mechanical transducers or interferometric optical sensors, detect the time, magnitude and shape of the arriving pressure waves and convert the stress profile into electrical signals that are received by an electronic tracer or scope device 102, for example, a digital oscilloscope that functions as an analog to digital converter and records the amplitude and temporal profile of the laser-induced stress waves. Signals from the electronic device 102, for example, digitized signals from the digital oscilloscope, are then analyzed by a computer 104 to reconstruct an image or representation 108 of the lesion on a graphical display 106. And, again, where the delay in the receive time of the acoustic waves is proportional to the distance of the sources generating the acoustic waves from the irradiation source, the detected signals can be used to determine tissue thickness, lesion progression, lesion width, and other assessment features in real time.

In accordance with the present invention, the stress detection of the illustrated embodiments of FIGS. 1 and 1A are accomplished in a reflection mode. And, in particular, with the catheter-based system of FIG. 1A, by integrating irradiation emission and optical detection in the catheter tip section 36, the stress waves detected have been reflected back toward the tissue surface that received the irradiation, where emphasis is made on high spatial resolution.

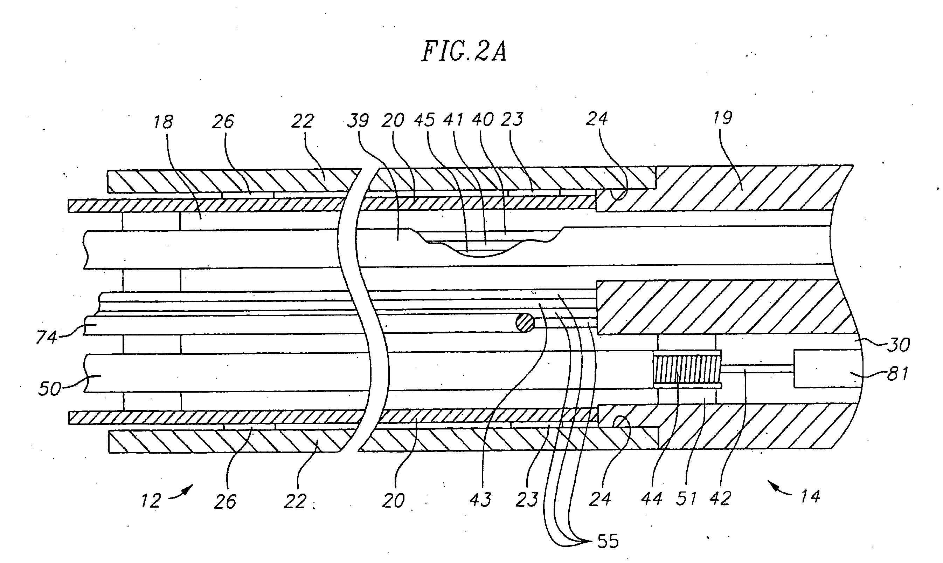

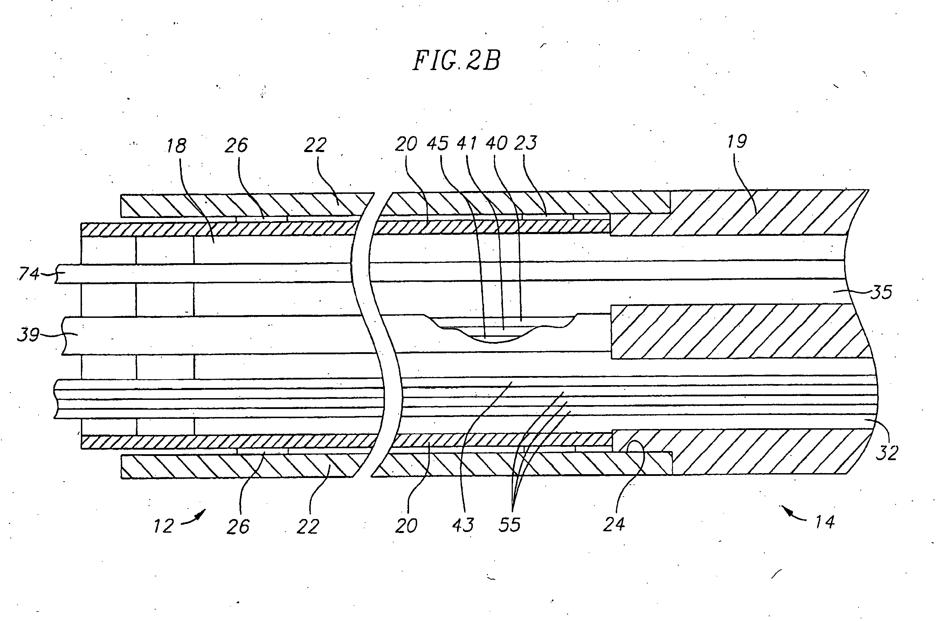

With reference to FIGS. 2A and 2B, an embodiment of a catheter 10 for use with the system S in accordance with the present invention comprises an elongated catheter body 12 having proximal and distal ends, a deflectable intermediate section 14 (uni or b-directionally) at the distal end of the catheter body 12, and a tip section 36 at the distal end of the intermediate section, and a control handle 16 at the proximal end of the catheter body 12. In accordance with the present invention, the tip section 36 incorporates an integrated design that provides both irradiation of the tissue of interest and detection of stress waves emanating therefrom.

The catheter body 12 comprises an elongated tubular construction having a single, axial or central lumen 18. The catheter body 12 is flexible, i.e., bendable, but substantially non-compressible along its length. The catheter body 12 can be of any suitable construction and made of any suitable material. A construction comprises an outer wall 22 made of an extruded plastic. The outer wall 22 may comprise an imbedded braided mesh of stainless steel or the like to increase torsional stiffness of the catheter body 12 so that, when the control handle 16 is rotated, the catheter body 12, the intermediate section 14 and the tip electrode 36 of the catheter 10 will rotate in a corresponding manner.

Extending through the single lumen 18 of the catheter body 12 are components, for example, an electrode lead wire 40 and thermocouple wires 41 and 45 protected by a sheath 39, a fiber optic cable 43, transducer lead wires 55, a compression coil 44 through which a puller wire 42 extends, and an electromagnetic sensor cable 74. A single lumen catheter body can be preferred over a multi-lumen body because it has been found that the single lumen body permits better tip control when rotating the catheter. The single lumen permits the various aforementioned components to float freely within the catheter body. If such components were restricted within multiple lumens, they tend to build up energy when the handle is rotated, resulting in the catheter body having a tendency to rotate back if, for example, the handle is released, or if bent around a curve, to flip over, either of which are undesirable performance characteristics.

The outer diameter of the catheter body 12 is not critical, but is preferably no more than about 8 french, more preferably 7 french. Likewise the thickness of the outer wall 22 is not critical, but is thin enough so that the central lumen 18 can accommodate the aforementioned components. The inner surface of the outer wall 22 may be lined with a stiffening tube 20, which can be made of any suitable material, such as polyimide or nylon. The stiffening tube 20, along with the braided outer wall 22, provides improved torsional stability while at the same time minimizing the wall thickness of the catheter, thus maximizing the diameter of the central lumen 18. The outer diameter of the stiffening tube 20 is about the same as or slightly smaller than the inner diameter of the outer wall 22. Polyimide tubing may be preferred for the stiffening tube 20 because it may be very thin walled while still providing very good stiffness. This maximizes the diameter of the central lumen 18 without sacrificing strength and stiffness.

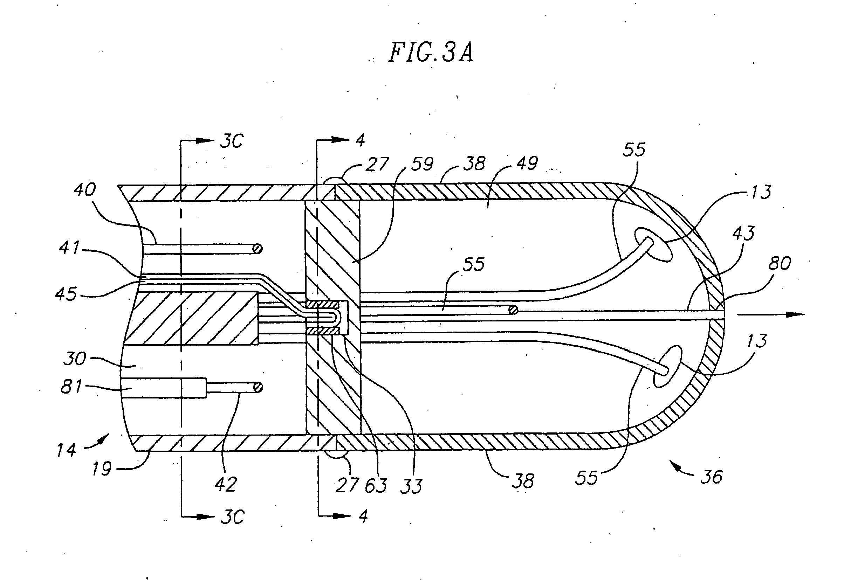

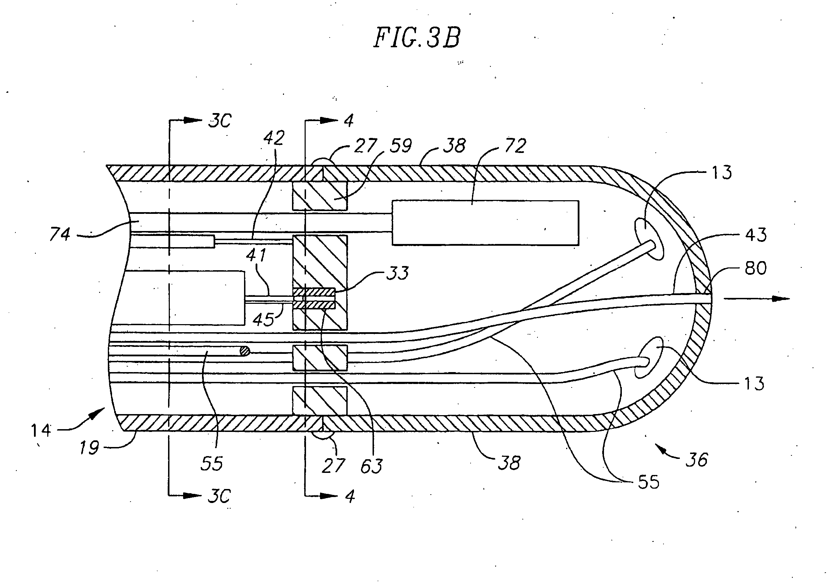

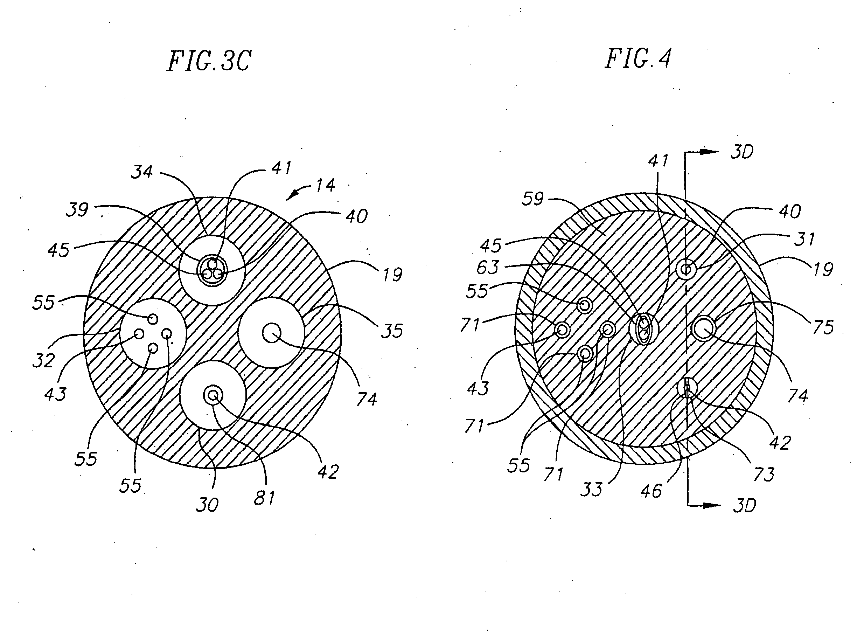

Referring also to FIGS. 3A, 3B and 3C, the intermediate section 14 comprises a shorter section of tubing 19 having multiple lumens. The tubing 19 is made of a suitable non-toxic material that is preferably more flexible than the catheter body 12. A suitable material for the tubing 19 is non-braided polyurethane. The outer diameter of the intermediate section 14, like that of the catheter body 12, is preferably no greater than about 8 french, more preferably 7 french. The size and number of the lumens is not critical. In an embodiment, the intermediate section 14 has an outer diameter of about 7 french (0.092 inch). The tubing has a first off-axis lumen 30, a second off-axis lumen 32, a third off-axis lumen 34 and a fourth off-axis lumen 35, that are generally about the same size, each having a diameter of from about 0.032 inch to about 0.038 inch, preferably 0.036 inch. In the illustrated embodiment, the puller wire 42 extends through the first lumen 30, and an optical waveguide, e.g., the fiber optic cable 43, and the transducer lead wires 55 extend through the second lumen 32. The electrode lead wire 40 extends through the third lumen 34. The thermocouple wires 41 and 45 also extend through the third lumen 34, and an electromagnetic sensor cable 74 extend through the fourth lumen 35.

As best shown in FIGS. 2A and 2B, the catheter body 12 in one embodiment is attached to the intermediate section 14 by means of an outer circumferential notch 24 configured in the proximal end of the tubing 19 that receives the inner surface of the outer wall 22 of the catheter body 12. The intermediate section 14 and catheter body 12 are attached by glue or the like. Before the intermediate section 14 and catheter body 12 are attached, the stiffening tube 20 is inserted into the catheter body 12. The distal end of the stiffening tube 20 is fixedly attached near the distal end of the catheter body 12 by forming a glue joint 23 with polyurethane glue or the like. Preferably a small distance, e.g., about 3 mm, is provided between the distal end of the catheter body 12 and the distal end of the stiffening tube 20 to permit room for the catheter body 12 to receive the notch 24 of the intermediate section 14. If no compression coil is used, a force is applied to the proximal end of the stiffening tube 20, and, while the stiffening tube 20 is under compression, a first glue joint (not shown) is made between the stiffening tube 20 and the outer wall 22 by a fast drying glue, e.g., cyanoacrylate. Thereafter a second glue joint 26 is formed between the proximal ends of the stiffening tube 20 and outer wall 22 using a slower drying but stronger glue, e.g., polyurethane.

If desired, a spacer can be located within the catheter body between the distal end of the stiffening tube and the proximal end of the tip electrode. The spacer provides a transition in flexibility at the junction of the catheter body and intermediate section, which allows this junction to bend smoothly without folding or kinking. A catheter having such a spacer is described in U.S. patent application Ser. No. 08/924,616, now US patent 5964757, entitled "Steerable Direct Myocardial Revascularization Catheter".

As illustrated in FIGS. 3A and 3B, the tip section 36 extends from the distal end of the intermediate section 14. In the illustrated embodiment, the tip electrode has a diameter about the same as the outer diameter of the tubing 19 of the intermediate section 14. The intermediate section 14 and the tip electrode are attached by glue 27 or the like applied circumferentially around a junction of the tubing 19 and the tip electrode 36. Moreover, the components extending between the intermediate section 14 and the tip electrode, e.g., the lead wire 40, the transducer lead wires 55, the thermocouple wires 41 and 45, and the puller wire 42, help keep the tip electrode on the intermediate section.

In the illustrated embodiment, the tip section 36 has a generally hollow distal portion. The tip electrode comprises a shell 38 of generally uniform thickness and a press-fit alignment member or plug 59 positioned at or near the proximal end of the shell to seal the hollow distal portion. The shell and the plug are formed from any suitable material that is both thermally and electrically conductive which allows for radio frequency ablation using an RF generator. Such suitable materials include, without limitation, platinum, gold alloy, or palladium alloy. A tip electrode and method for manufacturing same are disclosed in Application No. 11/058,434, filed February 14, 2005, published as US 2006/184165 A1 and Application No. 11/453,188; filed June 13, 2006, published as US 2007/287998 A1.

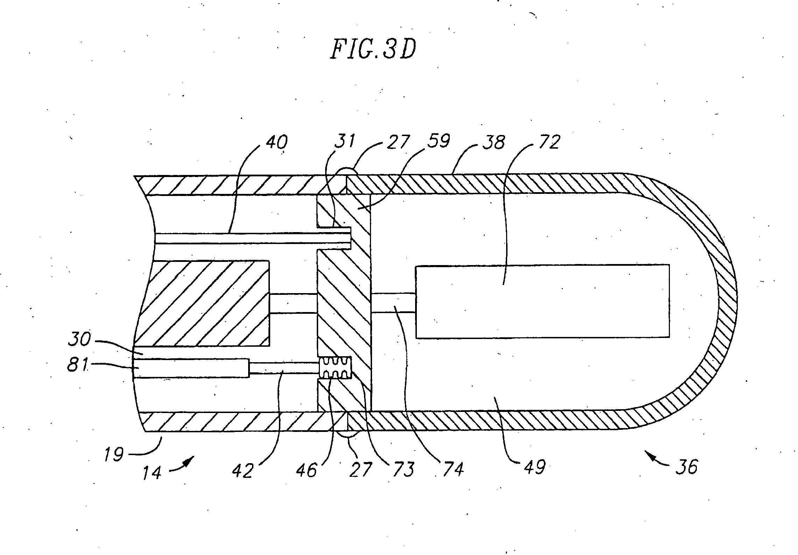

The tip section 36 is energized for RF ablation by the lead wire 40 that extends through the third lumen 34 of intermediate section 14, the central lumen 18 of the catheter body 12, and the control handle 16, and terminates at its proximal end in an input jack 75 that may be plugged into an appropriate monitor (not shown). The portion of the lead wire 40 extending through the central lumen 18 of the catheter body 12, control handle 16 and distal end of the intermediate section 14 is enclosed within the protective sheath 39, which can be made of any suitable material, preferably Teflon RTM. The protective sheath 39 is anchored at its distal end to the distal end of the intermediate section 14 by gluing it in the lumen 34 with polyurethane glue or the like. The lead wire 40 is attached to the tip electrode 36 by any conventional technique. In the illustrated embodiment, connection of the lead wire 40 to the tip section 36 is accomplished, for example, by welding the distal end of the lead wire 40 into a first blind hole 31 (FIG. 3D) in the alignment member 59 of the tip electrode 36.

A temperature sensing means is provided for the tip electrode 36 in the disclosed embodiment. Any conventional temperature sensing means, e.g., a thermocouple or thermistor, may be used. With reference to FIGS. 3A and 3B, a suitable temperature sensing means for the tip section 36 comprises a thermocouple formed by a wire pair. One wire of the wire pair is the copper wire 41, e.g., a number "40" copper wire. The other wire of the wire pair is the constantan wire 45, which gives support and strength to the wire pair. The wires 41 and 45 of the wire pair are electrically isolated from each other except at their distal ends where they contact and are twisted together, covered with a short piece of plastic tubing 63, e.g., polyimide, and covered with epoxy. The plastic tubing 63 is then attached in a second blind hole 33 of the tip electrode 36 (FIG. 3B), by epoxy or the like. The wires 41 and 45 extend through the third lumen 34 in the intermediate section 14. Within the catheter body 12 the wires 41 and 45 extend through the central lumen 18 within the protective sheath 39 along with the lead wire 40. The wires 41 and 45 then extend out through the control handle 16 and to a connector connectable to a temperature monitor (not shown). Alternatively, the temperature sensing means may be a thermistor. A suitable thermistor for use in the present invention is Model No. AB6N2-GC14KA143T/37C sold by Thermometrics (New Jersey).

Referring to FIGS. 2A, 3A and 3D, the puller wire 42 as part of a means for deflecting the catheter extends through the catheter body 12, is anchored at its proximal end to the control handle 16, and is anchored at its distal end to the tip electrode 36. The puller wire is made of any suitable metal, such as stainless steel or Nitinol, and is preferably coated with Teflon.RTM. or the like. The coating imparts lubricity to the puller wire. The puller wire preferably has a diameter ranging from about 0.006 to about 0.010 inches.

The compression coil 44 is situated within the catheter body 12 in surrounding relation to the puller wire. The compression coil 44 extends from the proximal end of the catheter body 12 to the proximal end of the intermediate section 14 (FIG. 2A). The compression coil is made of any suitable metal, preferably stainless steel, and is tightly wound on itself to provide flexibility, i.e., bending, but to resist compression. The inner diameter of the compression coil is preferably slightly larger than the diameter of the puller wire 42. The Teflon.RTM. coating on the puller wire allows it to slide freely within the compression coil. If desired, particularly if the lead wire 40 is not enclosed by a protective sheath, the outer surface of the compression coils can be covered by a flexible, non-conductive sheath, e.g., made of polyimide tubing, to prevent contact between the compression coils and any other wires within the catheter body 12.

As shown in FIG. 2A, the compression coil 44 is anchored at its proximal end to the proximal end of the stiffening tube 20 in the catheter body 12 by glue joint 50 and at its distal end to the intermediate section 14 by glue joint 51. Both glue joints 50 and 51 preferably comprise polyurethane glue or the like. The glue may be applied by means of a syringe or the like through a hole made between the outer surface of the catheter body 12 and the central lumen 18. Such a hole may be formed, for example, by a needle or the like that punctures the outer wall 22 of the catheter body 12 and the stiffening tube 20 which is heated sufficiently to form a permanent hole. The glue is then introduced through the hole to the outer surface of the compression coil 44 and wicks around the outer circumference to form a glue joint about the entire circumference of the compression coil.

With reference to FIGS. 2A, 3A and 3C, the puller wire 42 extends into the first lumen 30 of the intermediate section 14. The puller wire 42 is anchored at its distal end to the tip electrode 36 within the third blind hole 73 in the alignment member 59, as shown in FIG. 3D. A method for anchoring the puller wire 42 within the tip electrode 36 is by crimping metal tubing 46 to the distal end of the puller wire 42 and soldering the metal tubing 46 inside the blind hole 73. Anchoring the puller wire 42 within the alignment member 59 provides additional support, reducing the likelihood that the tip electrode 36 will fall off. Alternatively, the puller wire 42 can be attached to the side of the tubing 19 of the intermediate section 14 as understood by one of ordinary skill in the art. Within the first lumen 30 of the intermediate section 14, the puller wire 42 extends through a plastic, preferably Teflon.RTM., sheath 81, which prevents the puller wire 42 from cutting into the wall of the intermediate section 14 when the intermediate section is deflected.

Longitudinal movement of the puller wire 42 relative to the catheter body 12, which results in deflection of the tip electrode 36, is accomplished by suitable manipulation of the control handle 16. A suitable control handle is described in US Patent No. 6602242.

In the illustrated embodiment of FIGS. 3A, 3B and 3D, the tip section 36 carries an electromagnetic sensor 72. The electromagnetic sensor 72 is connected to the electromagnetic sensor cable 74, which extends through a passage 75 (FIG. 4) in the alignment member 39, the third lumen 35 of the tip electrode section 36 through the central lumen 18 of the catheter body 12, and into the control handle 16. As shown in FIG. 1, the electromagnetic sensor cable 74 then extends out the proximal end of the control handle 16 within an umbilical cord 78 to a sensor control module 75 that houses a circuit board (not shown). Alternatively, the circuit board can be housed within the control handle 16, for example, as described in U.S. Patent application Ser. No. 08/924,616, entitled "Steerable Direct Myocardial Revascularization Catheter", now US patent 5964757. The electromagnetic sensor cable 74 comprises multiple wires encased within a plastic covered sheath. In the sensor control module 75, the wires of the electromagnetic sensor cable 74 are connected to the circuit board. The circuit board amplifies the signal received from the electromagnetic sensor 72 and transmits it to a computer in a form understandable by the computer by means of the sensor connector 77 at the proximal end of the sensor control module 75, as shown in FIG. 1. Because the catheter can be designed for single use only, the circuit board may contain an EPROM chip which shuts down the circuit board approximately 24 hours after the catheter has been used. This prevents the catheter, or at least the electromagnetic sensor, from being used twice. Suitable electromagnetic sensors for use with the present invention are described, for example, in U.S. Pat. Nos. 5,558,091, 5,443,489, 5,480,422, 5,546,951, 5,568,809, and 5,391,199 and International Publication No. WO 95/02995. An electromagnetic mapping sensor 72 may have a length of from about 6 mm to about 7 mm and a diameter of about 1.3 mm.

In accordance with a feature of the present invention, the catheter 10 is adapted to facilitate optoacoustically-based real-time assessment of ablation tissue characteristics, including without limitation, tissue thickness, lesion progression, lesion width, and other assessment features in real time. These assessments are accomplished by employing non-resonant acoustic frequencies which are the result of optical absorption properties of the various tissue elements within the irradiation field of view of the catheter tip section. The catheter 10 therefore allows real-time assessment of lesion formation, tissue status and tissue morphology.

As shown in FIGS. 2A, 3A and 3B, an optical waveguide, e.g., the fiber optic cable 43 is provided in the catheter to emit irradiation at the distal end, whereby the light selectively absorbed by the lesion and surrounding tissue (solid and fluid medium) is converted to heat which produces an acoustic wave detectable by the transducers 13 integrated in the tip section 36. The fiber optic cable 43 transmits light from the light source 100 (FIG. 1) to the tip electrode 36. The fiber optic extends through the lumen 18 of the catheter body 12, through the second lumen 32 of the intermediate section 14 and into the tip section 36 where the distal end of the cable 43 is fixedly mounted in an on-axis irradiation opening 80 which is located generally at the most distal location along the longitudinal axis of the tip section 36 for on-axis transmission at the tip section. The fiber optic cable 43 may be any suitable optical wave guide wherein light introduced at one of the cable is guided to the other end of the cable with minimal loss. The cable 43 may be a single fiber optic cable or fiber bundles. It may be single mode (also known as mono-mode or uni--mode), multi-mode (with step index or graded index) or plastic optical fiber (POF), depending on a variety of factors, including but not limited to transmission rate, bandwidth of transmission, spectral width of transmission, distance of transmission, diameter of cable, cost, optical signal distortion tolerance and signal attenuation, etc.

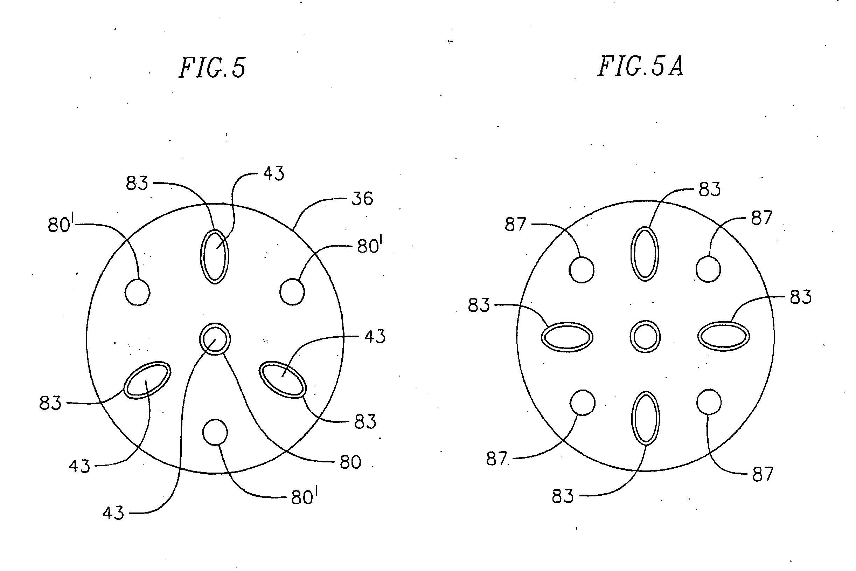

There are additional off-axis openings 83 formed in the tip section 36 in which the transducers 13 are mounted. In the illustrated embodiment of FIGS. 3, 3A, 3B and 5, there are three openings 83 for three respective transducers 13 that are arranged generally equi-spaced from each other and from the opening 80, and generally equi-angular about the opening 80, equally offset from each other at about 120 degrees. It is understood by one of ordinary skill in the art that the number and arrangement of the irradiation opening 80 and transducer openings 83 may be varied as appropriate or desired. For example, there can be off-axis irradiation openings 80' and/or additional transducer openings 83 (FIG. 5). The total number of openings 80' and 83 may range between about 3 to 6, where an embodiment with four openings could be arranged at about a 90 degree offset angle, five openings and transducers at about a 72 degree offset angle, or six openings and transducers at about a 60 degree offset angle.

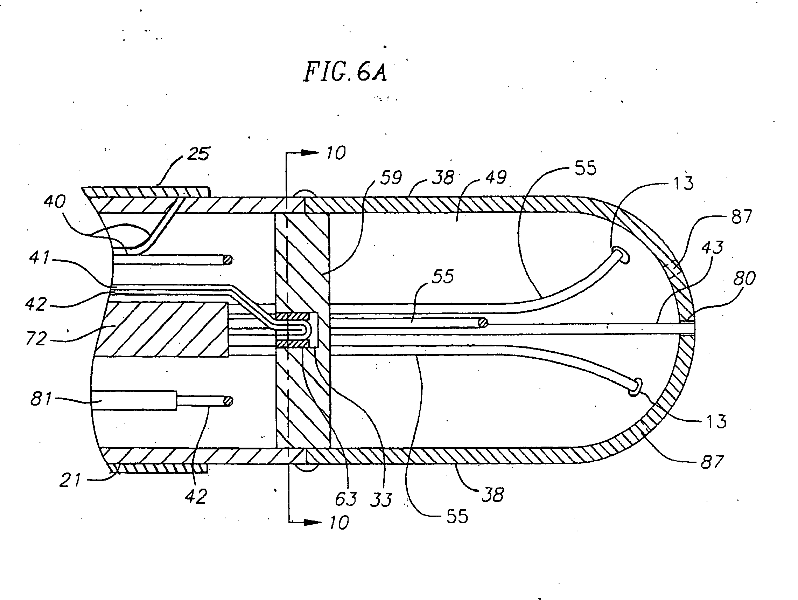

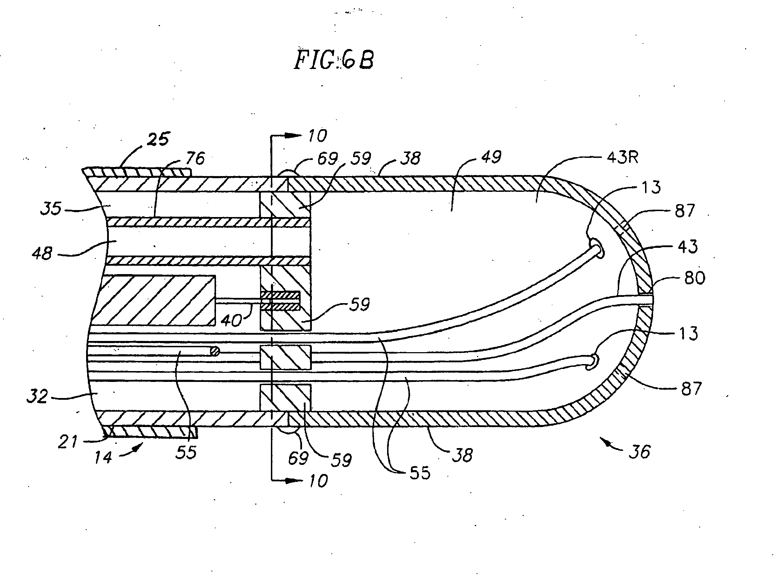

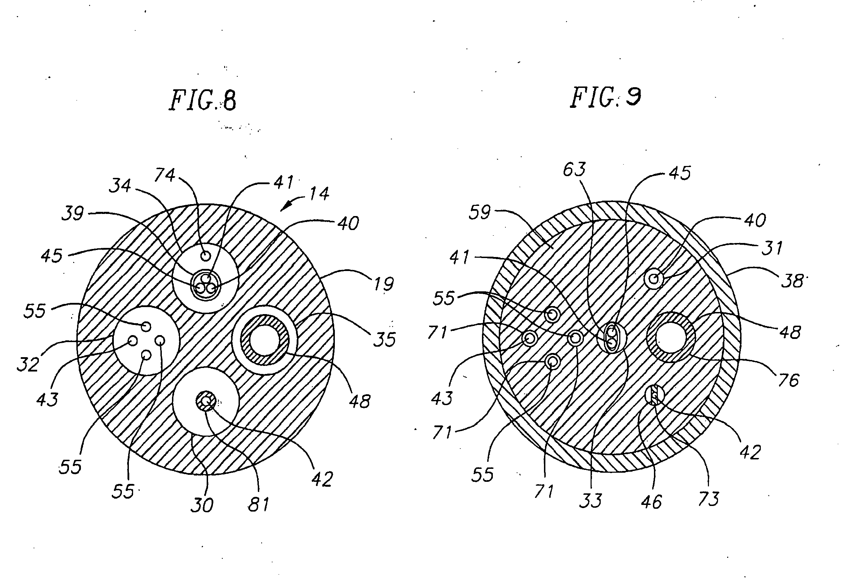

In the illustrated embodiment, the openings 80 and 83 are sized to receive the cable 43 and the transducers 13 in a generally snug-fit fashion. However, in an alternative embodiment as illustrated in FIGS. 6A and 6B, the openings 80, 80' are sized larger to allow fluid (e.g. saline) to flow pass the distal end of the cable(s) 43 and reach outside the tip electrode for cooling the tip electrode and ablation site and/or enabling larger and deeper lesions. Additional openings 87, as shown in FIG. 5A, may be formed in the shell to allow further irrigation of the tip electrode. The fluid is fed into the chamber 49 by an irrigation means, as shown in FIG. 6B, that include a tube segment 48 extending from the distal end of the fourth lumen 35 of the intermediate section 14 and a passage 76 in the plug 59 (FIG. 9). The distal end of the segment 48 is anchored in the passage 76 and the proximal end is anchored in the fourth lumen 35 by polyurethane glue or the like. Accordingly, the passage 76 is generally aligned with the fourth lumen 35 of the intermediate section 14. The segment 48, like the puller wires 42, provides additional support for the tip electrode. The irrigation tube segment 48 is in communication with a proximal infusion tube segment (not shown) that extends through the central lumen 18 of the catheter body 12 and terminates in the proximal end of the fourth lumen 35 of the intermediate section 14. The proximal end of the first infusion tube segment extends through the control handle 16 and terminates in a luer hub 90 (FIG. 1) or the like at a location proximal to the control handle. In practice, fluid may be injected by a pump (not shown) into the infusion tube segment through the luer hub 90, through the infusion tube segment 48, into the chamber 49 in the tip electrode 36, and out the openings 80. The infusion tube segments may be made of any suitable material, and is preferably made of polyimide tubing. A suitable infusion tube segment has an outer diameter of from about 0.32 inch to about 0.036 inch and an inner diameter of from about 0.28 inch to about 0.032 inch.

The pump can maintain the fluid at a positive pressure differential relative to outside the chamber 49 so as to provide a constant unimpeded flow or seepage of fluid outwardly from the chamber 49 which continuously seeps out from the openings 80.

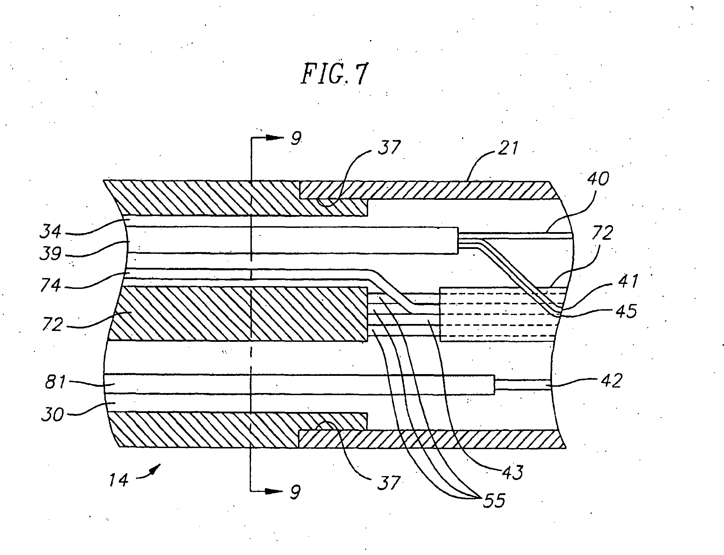

In the illustrated embodiment of FIGS. 6A, 6B and 7, a housing 21 extends between the intermediate section 14 and the tip electrode 36 so that the electromagnetic sensor 72 can remain near the tip electrode and remain dry. The housing 21 (e.g., a plastic tube member) is attached to the tubing 19 of the intermediate section by creating a circumferential notch 37 in the distal end of the tubing 19, placing the proximal end of the housing 21 on the distal end of the tubing 19, and filling the notch 37 with glue. The distal end of the housing 21 and the tip electrode 36 are attached by glue at a seam 69. All the components extending into or through the alignment member 59 help keep the tip electrode 36 attached to the housing 21.

As shown in FIG. 6A, the catheter may also be adapted for electrophysiologic mapping by providing ring electrodes 25 (unipolar or bipolar) proximal the tip electrode. In the illustrated embodiment, the ring electrodes are mounted on the intermediate section 14. The tip electrode and the ring electrodes are each connected to a separate lead wire 40. The lead wires 40 for the ring electrodes like the lead wire for the tip electrode extend through the lumen 34 of tip section 14, the central lumen 18 of the catheter body 12, and the control handle 16, and terminate at their proximal end in an input jack (not shown) that may be plugged into an appropriate signal processing unit (not shown) and source of RF energy (not shown). The distal end of the protective sheath 39 is proximal of the most proximal ring electrode thereby allowing the lead wire to connect to the ring electrode.

The lead wires 40 are attached to the ring electrode 25 by any conventional technique, for example, by making a hole through the side wall of the tubing 19. A lead wire 40 is then drawn through the hole and the ends of the lead wire 44 are then stripped of any coating and soldered or welded to the underside of the ring electrode 25, which is then slid into position over the hole and fixed in place with polyurethane glue or the like. The plurality, position and spacing of the ring electrode 59 are not critical. If desired, additional ring electrodes may be used and can be positioned over the flexible tubing 19 of the intermediate section 14 or the plastic housing 21 in a similar manner.

It is understood by one of ordinary skill in the art that any desired aspects of the different embodiments described herein may be incorporated within a catheter tip section so as to suit the needs and desires in a particular use and application. For example, the embodiment of FIGS. 6A, 6B and 7 need not include irrigation, but the EM sensor 72 can nevertheless be housed outside of the chamber 49, in tubing 21, especially if there is insufficient space in the chamber 49 to contain both the EM sensor 72, the fiber optic cable 43 and the transducer lead wires 55.

The system of the present invention may be used in a method of monitoring epi- or endo-cardial tissue with laser optoacoustic imaging. With reference to FIG. 1 and 1A, the method includes irradiating cardiac tissue from a distal end of a catheter to heat said tissue for producing an acoustic wave, detecting said acoustic wave with an acoustic transducer mounted on said distal end of the catheter, recording characteristics of the acoustic wave; and analyzing the acoustic wave to assess a tissue characteristic. In particular, the method includes the use of a catheter having an integrated distal end with both irradiation and detection capabilities, such that the acoustic detection is performed in a reflection mode which emphasizes high spatial resolution of the measured image.

The irradiation is selectively absorbed by the lesion and surrounding tissue and converted to heat. The heat produces an acoustic wave which is detected by the acoustic transducer or sensor. The delay in the receive time (or temporal profile) of the acoustic wave is proportional to the distance of the tissue elements generating the acoustic wave from the irradiating distal end of the catheter and is used to assess and determine in real time a variety of tissue characteristics, including the reconstruction of a cross-sectional image of the tissue monitored. The tissue characteristics that can be assessed, monitored or determined from the present invention includes, without limitation, tissue thickness, lesion progression, lesion width, and other assessment features. Tissue thickness can be determined in real time and up to a few centimeters with generally very high resolution. The method of the present invention employs non-resonant acoustic frequencies which are the result of optical absorption properties of the various tissue elements within the irradiation field of view of the catheter. The resulting signal typically has a much higher sensitivity to tissue elements with different optical absorption properties, such as those between tissue in various states of ablation, tissue and blood, etc.

The foregoing descriptions are directed to a catheter that is configured both for RF ablation and optoacoustic-based assessment from the distal end. As an alternative, the person skilled in the art might contemplate a diagnostic catheter that provides irradiation and acoustic detection from the distal end on a tissue of interest while a separate therapeutic catheter performs RF ablation on that tissue. The catheter may be nonirrigated or irrigated.

The present invention can be utilized to determine lesion boundaries in real-time as a lesion is being created in cardiac tissue via RF, ultrasound, laser cryotherapy, high intensity focused ultrasound (HIFU), or laser. In addition, the present invention can also determine tissue dimensions (thickness) in real time, concurrent with the lesion formation. The present invention can then be used to determine the progression of a lesion towards the distal tissue margin, and indicate transmurality when the lesion progresses through the entire tissue thickness. Moreover, in epi/endocardial applications, the present invention can also use a combination of catheters to allow a full range of detection combinations (inside, outside).

The preceding description has been presented with reference to presently preferred embodiments of the invention. Workers skilled in the art and technology to which this invention pertains will appreciate that alterations and changes in the described structure may be practiced without meaningfully departing from the scope of this invention. For example, laser pulses can be delivered via optical fibers, hollow or liquid waveguides, or free-space optics. All wavelengths transmittable by the delivery optics can be used for this technique. A variety of acoustic sensors can be used, including piezoelectric transducers, mechanical transducers, or interferometric optical sensors. The ablation element can include a variety of energy sources, including RF, ultrasound, cryotherapy, HIFU, or laser.

Accordingly, the foregoing description should not be read as pertaining only to the precise structures described and illustrated in the accompanying drawings, but rather should be read consistent with and as support to the following claims which are to have their fullest and fair scope.

## Claims

1. A system for opto-acoustic tissue assessment, comprising:
a catheter (10) having a distal tip section (36) configured for irradiation and acoustic detection, wherein the irradiation is adapted to heat a tissue to produce an acoustic wave detectable by an acoustic detector (13) mounted on the tip section and the acoustic detector is configured to generate a signal representative of a tissue characteristic;
an electronic scope (102) for receiving the signal to record a temporal profile of the acoustic wave; and
a processor configured to reconstruct an image or profile of the tissue based on the temporal profile;
**characterised in that**:
the tip section of the catheter is also adapted for RF ablation; and **in that** the system is configured to determine the width of a lesion resulting from RF ablation.

2. The system of claim 1, wherein the electronic scope device is a digital oscilloscope.

3. The system of claim 1, wherein the catheter operates in a reflection mode during the opto-acoustic tissue assessment.

4. The system of claim 1, further comprising a light source (100) to provide the irradiation as a laser pulse.

5. The system of catheter of claim 1, wherein the tissue characteristic further comprises at least one of the following: tissue thickness and lesion progression.

6. The system of claim 1, wherein the system is adapted to assess cardiac tissue.

## Patentansprüche

1. System zur optoakustischen Gewebebeurteilung, umfassend:
einen Katheter (10) mit einem distalen Spitzenabschnitt (36), der zur Bestrahlung und zur akustischen Erfassung konfiguriert ist, wobei die Bestrahlung geeignet ist, ein Gewebe zu erhitzen, so dass es eine akustische Welle erzeugt, die von einem akustischen Detektor (13) erfassbar ist, der am Spitzenabschnitt montiert ist, wobei der akustische Detektor dazu konfiguriert ist, ein eine Gewebeeigenschaft repräsentierendes Signal zu erzeugen,
eine elektronische Anzeigevorrichtung (102) zum Empfangen des Signals, um einen zeitlichen Verlauf der akustischen Welle aufzuzeichnen, und
einen Prozessor, der dazu konfiguriert ist, ein Bild oder Profil des Gewebes auf der Grundlage des zeitlichen Verlaufs zu rekonstruieren,
**dadurch gekennzeichnet, dass**
der Spitzenabschnitt des Katheters auch zur HF-Ablation geeignet ist und dass das System dazu konfiguriert ist, die Breite einer sich durch HF-Ablation ergebenden Läsion zu bestimmen.

2. System nach Anspruch 1, wobei die elektronische Anzeigevorrichtung ein digitales Oszilloskop ist.

3. System nach Anspruch 1, wobei der Katheter während der optoakustischen Gewebebeurteilung in einem Reflexionsmodus arbeitet.

4. System nach Anspruch 1, ferner umfassend eine Lichtquelle (100) zur Bereitstellung der Bestrahlung als ein Laserimpuls.

5. System nach Anspruch 1, wobei die Gewebeeigenschaft ferner mindestens eine der folgenden umfasst: Gewebedicke und Fortschreiten der Läsion.

6. System nach Anspruch 1, wobei das System geeignet ist, Herzgewebe zu beurteilen.

## Revendications

1. Système d'évaluation opto-acoustique de tissus, comprenant :
un cathéter (10) ayant une section de pointe distale (36) configurée pour l'irradiation et la détection acoustique, l'irradiation étant prévue pour chauffer un tissu de manière à produire une onde acoustique pouvant être détectée par un détecteur acoustique (13) monté sur la section de pointe et le détecteur acoustique étant configuré pour générer un signal représentant une caractéristique du tissu ;
une sonde électronique (102) pour recevoir le signal pour enregistrer un profil temporel de l'onde acoustique ; et
un processeur configuré pour reconstruire une image ou un profil du tissu sur la base du profil temporel ;
**caractérisé en ce que**
la section de pointe du cathéter est également prévue pour une ablation RF ; et **en ce que** le système est configuré pour déterminer la largeur d'une lésion résultant de l'ablation RF.

2. Système selon la revendication 1, dans lequel le dispositif de sonde électronique est un oscilloscope numérique.

3. Système selon la revendication 1, dans lequel le cathéter fonctionne en mode de réflexion au cours de l'évaluation opto-acoustique des tissus.

4. Système selon la revendication 1, comprenant en outre une source de lumière (100) pour fournir l'irradiation sous forme d'impulsions laser.

5. Système de cathéter selon la revendication 1, dans lequel la caractéristique du tissu comprend en outre au moins l'une des caractéristiques suivantes : l'épaisseur du tissu et la progression de la lésion.

6. Système selon la revendication 1, dans lequel le système est prévu pour évaluer des tissus cardiaques.
